# EUROPEAN PATENT SPECIFICATION

(11) **EP 0 647 229 B1**
(45) Date of publication and mention of the grant of the patent: **24.07.1996**
(21) Application number: 94911952.3
(22) Date of filing: 22.03.1994
(51) Int. Cl.: C07D 503/00, A61K 31/42

(54) **DIAMINE SALTS OF CLAVULANIC ACID**
DIAMINSALZE VON CLAVULANSÄURE
SELS DIAMINE D'ACIDE CLAVULANIQUE

(30) Priority: 26.03.1993 EP 93200872
(43) Date of publication of application: 12.04.1995
(60) Divisional application: 96200597.1
(73) Proprietor: GIST-BROCADES N.V., NL-2600 MA Delft (NL)
(72) Inventor: WEBER, Pieter, Gijsbert, NL-2985 BN Ridderkerk (NL)
(74) Representative: Visser-Luirink, Gesina, Dr.
(86) International application number: PCT/EP94/00919
(87) International publication number: WO 94/22873

(56) References cited:
- EP-A- 0 026 044
- EP-A- 0 387 178
- EP-A- 0 562 583

## Description

The present invention relates to new diamine salts of clavulanic acid, pharmaceutical compositions thereof, and to the use of these salts in the production of clavulanic acid and salts and esters thereof.

GB patent 1508977 discloses that clavulanic acid, which has the formula (I): 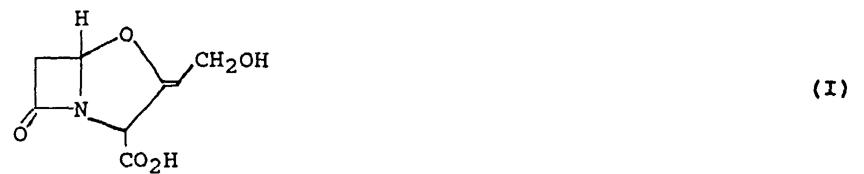 and its pharmaceutically acceptable salts and esters are antibacterial agents, able to enhance the effectiveness of penicillins and cephalosporins against many β-lactamase-producing bacteria.

US patent 4,650,795 discloses a group of primary amine salts of clavulanic acid which give stable pharmaceutical compositions.

EP patent 26044 discloses the use of the t-butylamine salt as a useful intermediate in the preparation of clavulanic acid.

The non-prepublished EP patent application 562583 discloses the same use for N,N-diisopropylethylenediammonium di clavulanate and N,N-diethylethylenediammonium di clavulanate, both of the secondary, secondary type.

Surprisingly it has been found that tertiary, tertiary diamine salts of clavulanic acid have improved properties compared to the t-butyl amine salt of clavulanic acid mentioned above. For instance, large crystals of the mono salt of N,N,N',N'-tetramethyl-1,2-diaminoethane clavulanate can easily be precipitated in pure form. The salt is therefore a very useful intermediate in the preparation of clavulanic acid.

Accordingly, the present invention provides tertiary, tertiary diamine mono salts of the formula (IIa): 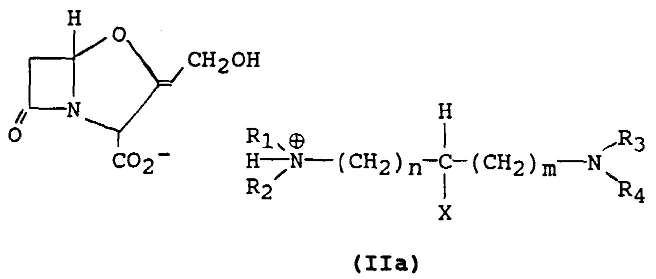 where R₁ and R₂ are each a (1-8C)alkyl, (3-8C)cycloalkyl or (3-8C)cycloalkyl(1-8C)alkyl group, optionally having one or more inert substituents selected from the group consisting of halogen, hydroxy, C₁ to C₄ alkyl, C₁ to C₄ alkoxyl, C₁ to C₄ acyloxyl and C₁ to C₄ esterified carboxyl, or are interlinked to form a ring of 4-7 ring atoms; R₃ and R₄ are each a (1-8C) alkyl, (3-8C)cycloalkyl or (3-8C)cycloalkyl(1-8C)alkyl group, optionally having one or more inert substituents selected from the group consisting of halogen, hydroxy, C₁ to C₄ alkyl, C₁ to C₄ alkoxyl, C₁ to C₄ acyloxyl and C₁ to C₄ esterified carboxyl, or are interlinked to form a ring of 4-7 ring atoms; X is hydrogen, hydroxy or halogen; and m and n are each 0-5, respectively.

An alkyl group may be branched or straight chain.

A C₁ to C₈ alkyl group is preferably a C₁ to C₄ alkyl group, for example, methyl, ethyl, propyl, isopropyl, butyl, sec.butyl or tert.butyl, more preferably it is methyl.

A C₃ to C₈ cycloalkyl group is preferably a C₅ to C₇ cycloalkyl group, for example cyclopentyl, cyclohexyl or cycloheptyl.

Preferably, X is hydrogen, hydroxy or halogen, for instance bromine or chlorine. Most preferably, X is hydrogen or hydroxy.

Preferably n is from 0 to 3 and m is from 0 to 3, more preferably when X is hydrogen or hydroxy. Most preferably n = 1, m is 0 and X is hydrogen or n is 1, m is 1 and X is hydroxy.

Suitably inert substituents include halogen, hydroxy, C₁ to C₄ alkyl, C₁ to C₄ alkoxyl, C₁ to C₄ acyloxyl and C₁ to C₄ esterified carboxyl.

A halogen atom is, for example, bromine, chlorine or fluorine, preferably, bromine or chlorine.

A C₁ to C₄ alkyl is preferably methyl or ethyl.

A C₁ to C₄ alkoxyl is preferably methoxyl or ethoxyl.

A C₁ to C₄ acyloxyl is preferably C₁ or C₂ acyloxyl.

A C₁ to C₄ esterified carboxyl is preferably C₁ or C₂ esterified carboxyl.

Generally, R₁, R₂, R₃ and R₄ have three substituents or fewer, preferably two substituents or fewer. Most preferably R₁, R₂, R₃ and R₄ have one substituent or are unsubstituted.

When R₁ and R₂ or R₃ and R₄ are interlinked to form a ring of 4 to 7 atoms, the ring consists preferably of carbon atoms and is most preferably saturated. Most preferably R₁, R₂, R₃ and R₄ are methyl.

Normally the amine of the formula (III): 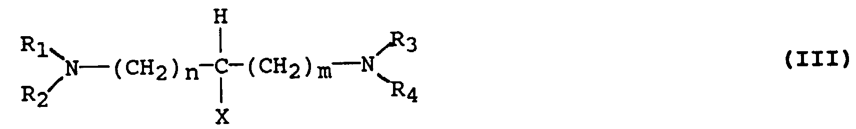 from which the salts of the formula (IIa) are derivable is a pharmaceutically acceptable amine.

Preferably, the salts of the formula (IIa) are derivable from N,N,N',N'-tetramethyl-1,2-diaminoethane, 1,3-bis(dimethylamino)-2-propanol, N,N,N',N'-tetramethyl-1,4-diaminobutane, N,N,N',N'-tetramethyl-1,6-diaminohexane, 1,2-dipiperidinoethane and dipiperidinomethane.

The present invention also provides a process for the preparation of a salt of formula (IIa) which process comprises the reaction of clavulanic acid with diamine (III): 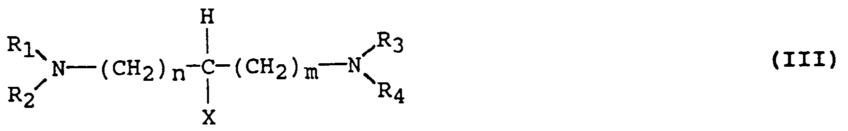 where R₁, R₂, R₃, R₄, X, m and n are as above defined. A diamine mono clavulanate will be formed when the amount of diamine is relatively high compared to that of clavulanic acid and the diamine di clavulanate will be formed when the amount of diamine is relatively low compared to that of clavulanic acid or a mixture of the same at a concentration in between.

The conditions when mono or diamine salts of clavulanic acid or a mixture of the same will be formed have not been investigated for each diamine, but it will be clear for someone skilled in the art that these will vary with the diamine applied.

The concentration of diamine present in the reaction mixture may be varied by, for example, varying the pH. At relatively high pH (dependent on the amine and the solvent used) more mono-protonated diamine (IIIa): 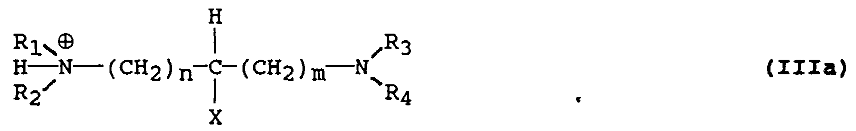 will be present and therefore more mono salt will be precipitated, and at relatively low pH more di-protonated diamine (IIIb) : 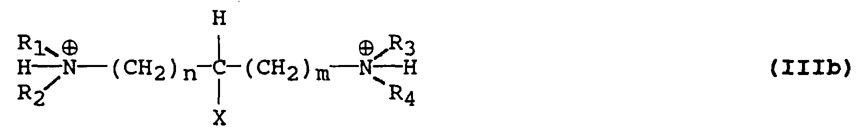 will be present and therefore more di salt will be precipitated.

The present invention further provides the use of diamine salts of clavulanic acid as defined above as an intermediate in the preparation of clavulanic acid and a pharmaceutically acceptable salt or ester thereof.

In another aspect the present invention provides a process for the preparation of clavulanic acid or a pharmaceutically acceptable salt or ester thereof which process comprises converting a diamine salt of clavulanic acid as defined above into clavulanic acid, generally by acidification, or a pharmaceutically acceptable salt or ester thereof, generally by adding a source of corresponding salt or ester forming compound.

In a further aspect the present invention provides a process for the purification of clavulanic acid or a pharmaceutically acceptable salt or ester thereof which process comprises:
i) contacting impure clavulanic acid in an organic solvent with diamine to form a salt of formula (IIa);
ii) isolating the salt produced in step i); and
iii) converting the isolated salt into clavulanic acid, generally by acidification, or a pharmaceutically acceptable salt or ester thereof generally by adding a source of a corresponding salt or ester forming compound. For instance, potassium clavulanate is formed by adding potassium acetate or potassium ethylhexanoate.

Most suitably the formation of the diamine salts of clavulanic acid takes place in an organic solvent. Suitable solvents include non-hydroxylic solvents such as, for example, tetrahydrofuran, dioxane, ethyl acetate, methyl acetate, acetone, methylethylketone and the like solvent and mixtures thereof.

The reaction may be carried out at from about -50°C to 40°C, most preferably from about 0°C to 15°C.

The present invention also provides pharmaceutical compositions which comprise a salt of the formula (IIa) and a pharmaceutically acceptable carrier.

Suitable forms of the compositions of this invention include tablets, capsules, reconstitutable powders and sterile forms suitable for injection or infusion. Such compositions may contain conventional pharmaceutically acceptable materials such as diluents, binders, colours, flavours, preservatives and disintegrants.

Injectable or infusible compositions of the salts of formula (IIa) are particularly suitable as high tissue levels of the compound of clavulanic acid can occur after administration by injection or infusion. Thus, one preferred composition aspect of this invention comprises salts of the formula (IIa) in sterile form.

Unit dose compositions comprising a salt of the formula (II) adapted for oral administration form a further preferred composition aspect of this invention.

The following examples will illustrate the invention. The pH value mentioned relates to this value measured with an Ingold electrode, type U402-S7/120, in the solvents applied.

### Examples

### Example 1

### Comparison of crystallization of various diamine salts

A solution of potassium clavulanate in ice cold water was stirred with ethyl acetate under cooling with ice-water. With a solution of about 10% (w/w) sulphuric acid the pH was brought at about 2, the water layer separated and twice extracted with ethyl acetate. The collected extracts were dried with magnesium sulphate, filtered and washed with ethyl acetate, yielding a solution of clavulanic acid of about 2% (w/w). The clavulanic acid was diluted with an equal volume of acetone and diamine was added.

The results are summarized in Table I. The ratio moles diamine/moles clavulanic acid has been indicated in the last column. The tertiary, tertiary type diamine salts of clavulanic acid are present in the preferred crystal form only, viz. not in the form of an oil.

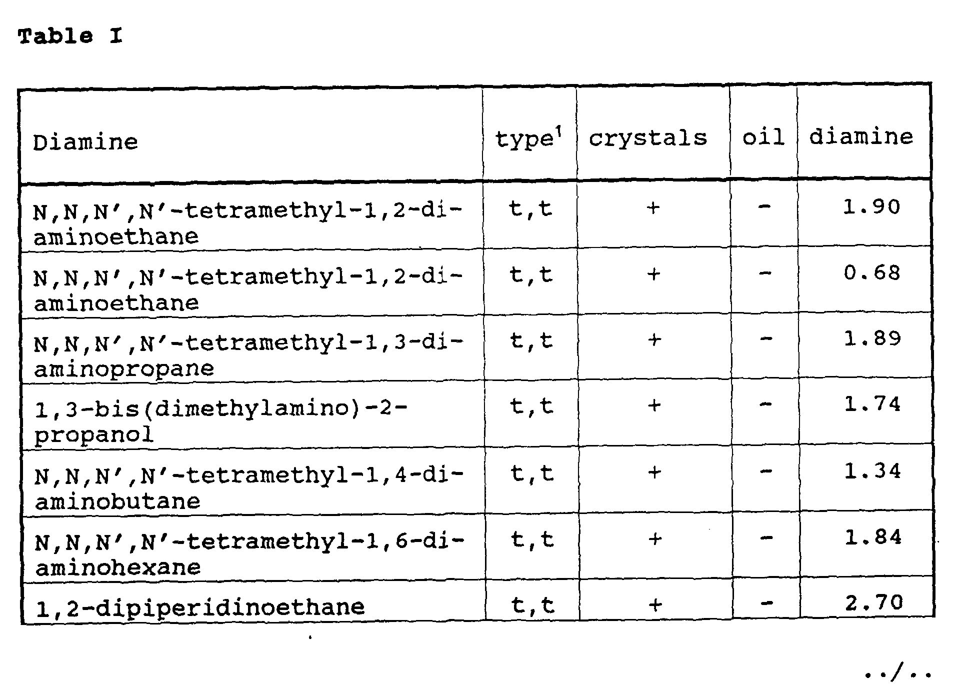

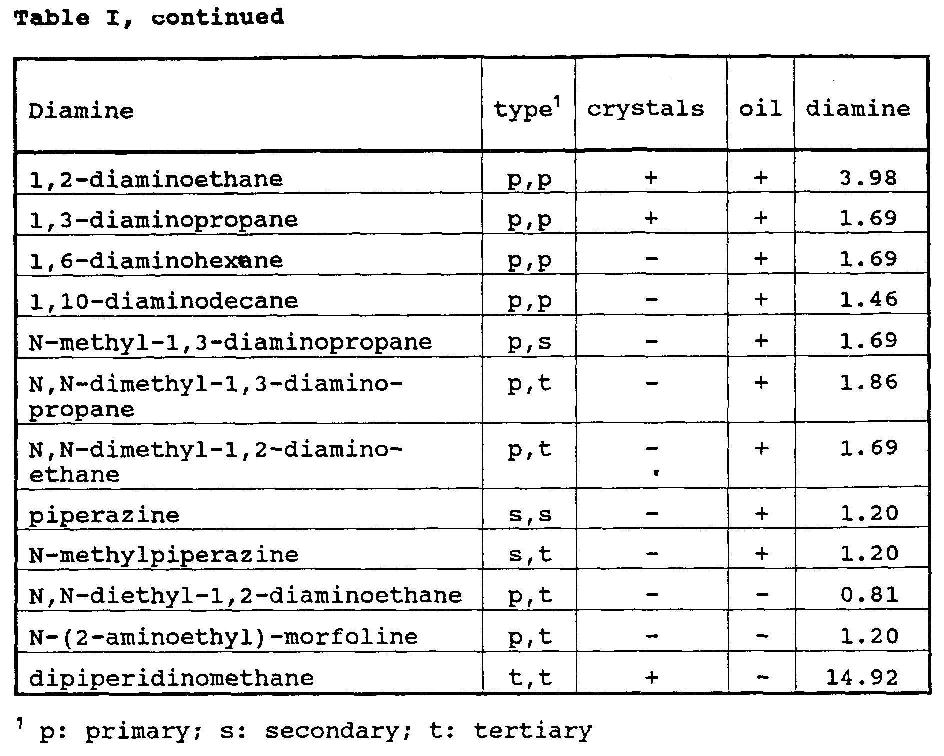

### Example 2

### Preparation of N,N,N',N'-tetramethyl-1,2-diaminoethane mono clavulanate from ethyl acetate/acetone solution

A clavulanic acid extract in dry ethyl acetate (828 g containing 18 g of clavulanic acid/kg prepared according to example 1) was added in 20 min to 1 l of acetone while cooling (8°C) and keeping the pH at 9 with N,N,N',N'-tetramethyl-1,2-diaminoethane (TMEDA, 19.18 g, viz. a molar ratio of 2.2 related to clavulanic acid). Stirring was continued at 10°C for 1 hr. The precipitate was filtered off and washed with 100 ml of acetone and dried in vacuum at 35°C to yield 20.09 g of TMEDA mono clavulanate (large crystals). The mother liquor (1642 g) contained about 1.12 g of clavulanic acid.

NMR (DMSO-d6) : 2.37 ppm, N-CH₃ (s, 12H); 2.70 ppm, N-CH₂ (s, 4H); 2.95 ppm, C6-βH (d, 1H); 3.49 ppm, C6-αH (dd, 1H); 3.99 ppm, CH₂OH (m, 2H); 4.60 ppm, C3-H (s, 1H); 4.66 ppm, =C-H (tr, 1H); 5.58 ppm, C5-H (d, 1H).

To 1 g of this TMEDA mono clavulanate 200 ml of a mixture of ethyl acetate/acetone (1/1 v/v) comprising 1 ml of TMEDA was added. After stirring during 1 hr at room temperature and filtrating the solution, the filtrate was slowly evaporated, resulting in large crystals.

All parameters were obtained by least squares from 2 e values for reflections measured on a diffractometer under the following experimental conditions:
- CAD 4 Rf Nonius
- Θ = 30°
- Mo-Kα radiation
- λ = 0.70145 Å.
- The salt (C₁₄H₂₅N₃O₅, group 1 C₈H₉NO_{S} and group 2 C₆H₁₆N₂; Mw = 315.37) crystallizes in the orthorhombic space group P2₁2₁2₁ with a = 8.268(1), b = 9.929(7) and c = 20.221(2) Å. α, β and γ = 90°.
- R = 0.206 for 2747 reflections.
- Z = 4.
- The molecules are bonded via a strong hydrogen bond (2.65(1) Å) between N(2) and 0(3). The C(6)-O(2) and C(6) -O(3) distances are 1.20(1) and 1.275(9) A, respectively, indicating a partial single bond character between C(6)-O(3).

The atom coordinates are shown in Table II:

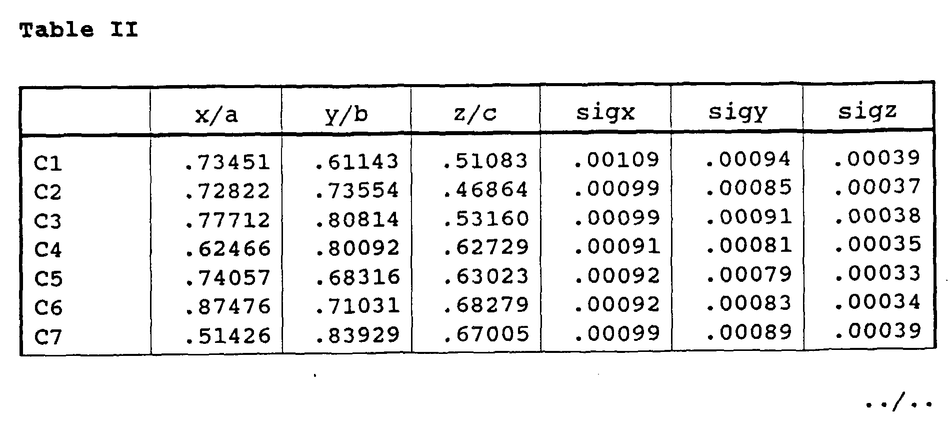

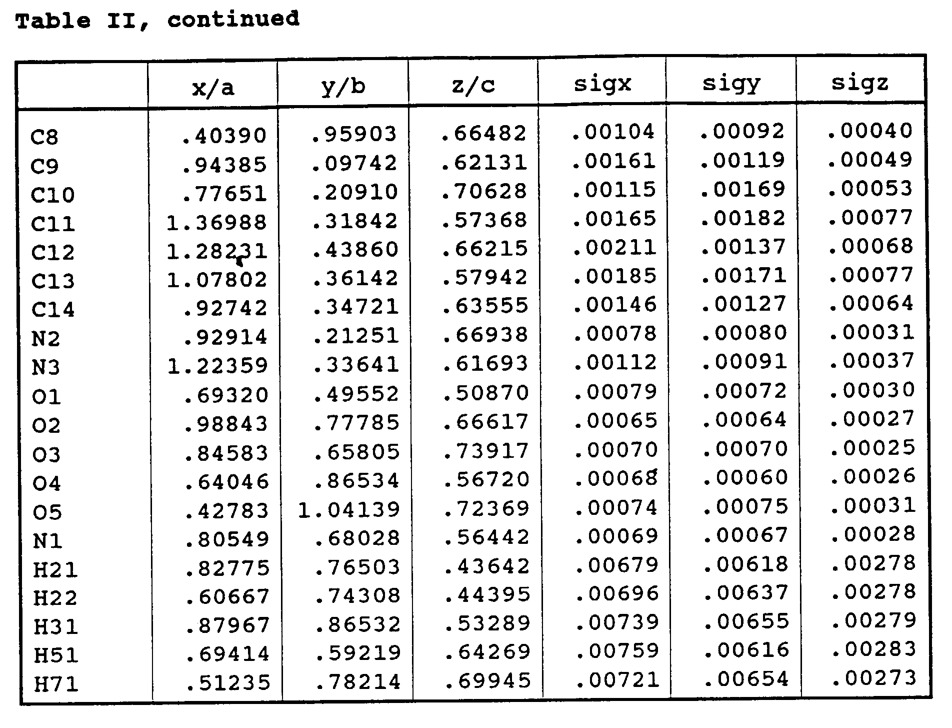

### Example 3

### Preparation of N,N,N',N'-tetramethyl-1,2-diaminoethane mono clavulanate from ethyl acetate solution

A solution of clavulanic acid in ethyl acetate (75 g, containing about 20 g of clavulanic acid/kg) was added in 10 min to 75 ml of ethyl acetate at 8°C while stirring and keeping the pH between 8 and 9 with N,N,N',N'-tetramethyl-1,2-diaminoethane (TMEDA, 5.11 g, viz. a molar ratio of 8 related to clavulanic acid). Stirring was continued for 0.5 hr and the precipitate was filtered off, washed with ethyl acetate and dried in vacuum at 35°C to give 2.62 g of TMEDA mono clavulanate (large crystals). The mother liquor (155 g) contained 0.03 g of clavulanic acid.

NMR (DMSO-d6): 2.38 ppm, N-CH₃ (s, 12H); 2.70 ppm, N-CH₂ (s, 4H); 2.93 ppm, C6-βH (d, 1H); 3.48 ppm, C6-αH (dd, 1H); 3.98 ppm, CH₂OH (m, 2H); 4.58 ppm, C3-H (s, 1H); 4.65 ppm, =C-H (tr, 1H); 5.58 ppm, C5-H (d, 1H).

### Example 4

### Preparation of 1,3-bis(dimethylamino)-2-propanol mono clavulanate

A solution of clavulanic acid in ethyl acetate (100 g, containing 20 g clavulanic acid/kg) was added over a period of 10 min to 100 ml of acetone while stirring at 8°C and keeping the pH between 8.5 and 8.7 with 1,3-bis(dimethylamino)-2-propanol (2.54 g, viz. a molar ratio of 1.74 related to clavulanic acid). Stirring was continued for 0.25 hr and the precipitate was filtered off and washed with 50 ml of a 1/1 mixture of acetone and ethyl acetate and with acetone. Drying in vacuum at room temperature yielded 1.82 g of 1,3-bis(dimethylamino)-2-propanol mono clavulanate with a purity of 25.6% as free acid.

NMR (DMSO-d6): 2.41 ppm, N-CH₃ (s, 12H); 2.50 ppm, N-CH₂ (dABq, 4H, J 12.6 Hz, J 4.8 Hz); 2.61 ppm, N-CH₂ (dABq, 4H, J 12.6 Hz, J 4.8 Hz) ; 2.94 ppm, C6-βH (d, 1H, J 16.5 Hz) ; 3.49 ppm, C6-αH (dd, 1H, J 16.5 Hz, J 2,7 Hz); 3.96 ppm CH₂OH, CHOH (m, 3H); 4.58 ppm, C3-H (s, 1H); 4.64 ppm =C-H (tr, 1H, J 6.8 Hz); 5.57 ppm, C5-H (d, 1H, J 2.6 Hz).

### Example 5

### Conversion of N,N,N',N'-tetramethyl-1,2-diaminoethane mono clavulanate into potassium clavulanate

30 ml of a 0.35 M potassium acetate solution (solvent isopropylalcohol and 1% (w/v) water) was added dropwise to a suspension of 2 g of N,N,N',N'-tetramethyl-1,2-diamminoethane mono clavulanate (content 68.6%) in 50 ml of isopropanol. After 0.75 hr stirring at room temperature the precipitate was filtered, washed with 10 ml of isopropanol and dried in vacuum at 35°C yielding 1.44 g of crystalline potassium clavulanate with a content of 87% (clavulanic acid) and about 1.5% potassium acetate (HPLC analysis). The mother liquor contained about 0.06 g of clavulanic acid.

### Example 6

### Conversion of 1,3-bis(dimethylamino)-2-propanol mono clavulanate into potassium clavulanate

1.6 ml of a 2M solution of potassium 2-ethyl-hexanoate in isopropanol was added to a stirred solution of 1 g of 1,3-bis(dimethylamino) -2-propanol mono clavulanate in 19 ml of isopropanol and 1 ml of water at room temperature. After stirring for 0.25 hr the precipitate was filtered off and washed with 15 ml of isopropanol. Drying in vacuum at room temperature gave 0.38 g of potassium clavulanate with a purity of 80% as free acid.

## Claims

1. A salt of clavulanic acid of the formula (IIa): 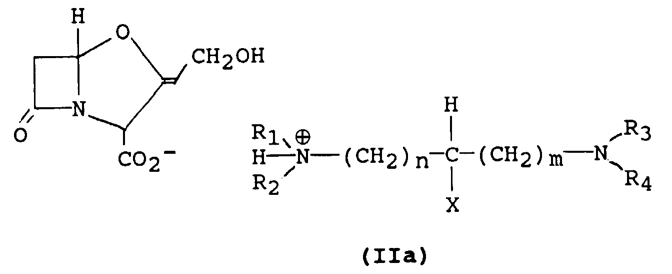 where R₁ and R₂ are each a (1-8C)alkyl, (3-8C)cycloalkyl or (3-8C)cycloalkyl(1-8C)alkyl group, optionally having one or more inert substituents selected from the group consisting of halogen, hydroxy, C₁ to C₄ alkyl, C₁ to C₄ alkoxyl, C₁ to C₄ acyloxyl and C₁ to C₄ esterified carboxyl, or are interlinked to form a ring of 4-7 ring atoms; R₃ and R₄ are each a (1-8C)alkyl, (3-8C)cycloalkyl or (3-8C)cycloalkyl(1-8C)alkyl group, optionally having one or more inert substituents selected from the group consisting of halogen, hydroxy, C₁ to C₄ alkyl, C₁ to C₄ alkoxyl, C₁ to C₄ acyloxyl and C₁ to C₄ esterified carboxyl, or are interlinked to form a ring of 4-7 ring atoms; X is hydrogen, hydroxy or halogen; and m and n are each 0-5, respectively.

2. A salt of clavulanic acid according to claim 1 where R₁, R₂, R₃ and R₄ are methyl.

3. A salt of clavulanic acid according to claim 1 or 2 where X is hydrogen, n is 1 and m is 0.

4. A salt of clavulanic acid according to claim 1 or 2, wherein X is hydroxy, n is 1 and m is 1.

5. A salt of clavulanic acid of formula IIa according to claim 1, wherein R₁, R₂, R₃ and R₄ are methyl, X is hydrogen, n is 1 and m is 0.

6. A process for preparing a salt as claimed in claim 1, 2, 3 or 4, wherein clavulanic acid is reacted with a diamine (III): 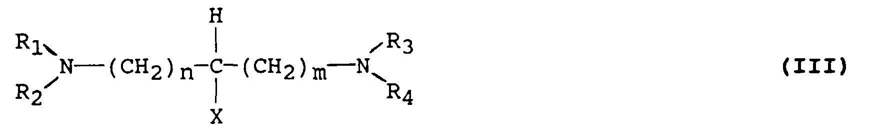 wherein R₁, R₂, R₃, R₄, X, m and n are as defined in claim 1, 2, 3 or 4, in an organic solvent.

7. A process according to claim 6 wherein the molar amount of diamine is greater than that of clavulanic acid.

8. A process to prepare a salt as claimed in claim 5, wherein clavulanic acid is reacted with N,N,N',N'-tetramethyl-1,2-diaminoethane wherein the molar amount of N,N,N',N'-tetramethyl-1,2-diaminoethane is greater than that of clavulanic acid.

9. A process for the preparation of clavulanic acid or a pharmaceutically acceptable salt or ester thereof which comprises converting a salt as claimed in claim 1, 2, 3, 4 and 5 into clavulanic acid or a pharmaceutically acceptable salt or ester thereof.

10. A process for the purification of clavulanic acid or a pharmaceutically acceptable salt or ester thereof which process comprises:
i) containing impure clavulanic acid in an organic solvent with diamine of formula (III) to form a salt of formula (IIa);
ii) isolating the salt produced in step i); and
iii) converting the isolated salt into clavulanic acid or a pharmaceutically acceptable salt or ester thereof.

11. A pharmaceutical composition comprising a salt of clavulanic acid as claimed in claim 1, 2, 3, 4 or 5 and a pharmaceutically acceptable carrier or diluent.

12. Use of a salt as claimed in claim 1, 2, 3, 4 or 5 as an intermediate in a process for the preparation of clavulanic acid or a pharmaceutically acceptable salt or ester thereof.

## Patentansprüche

1. Salz der Clavulansäure der Formel (IIa) 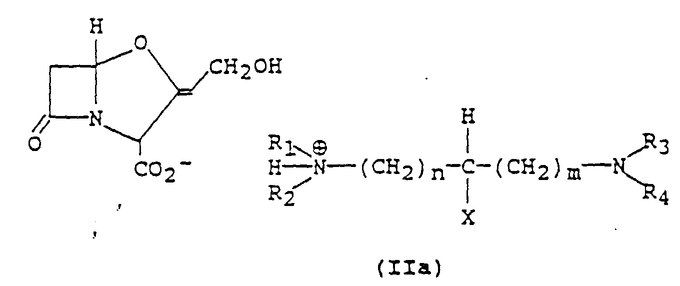 in der R₁ und R₂ jeweils einen C₁₋₈-Alkyl-, C₃₋₈-Cycloalkyl- oder C₃₋₈-Cycloalkyl-C₁₋₈-alkylrest, gegebenenfalls mit einem oder mehreren inerten Substituenten, bedeuten, wobei die Substituenten aus der Gruppe ausgewählt sind, die aus Halogen und einem Hydroxyl-, C₁₋₄-Alkyl-, C₁₋₄-Alkoxy-, C₁₋₄-Acyloxy- und C₁₋₄-Carboxylesterrest besteht, oder R₁ und R₂ zur Bildung eines Rings mit vier bis sieben Ringatomen miteinander verbunden sind, R₃ und R₄ jeweils einen C₁₋₈-Alkyl-, C₃₋₈-Cycloalkyl- oder C₃₋₈-Cycloalkyl-C₁₋₈-alkylrest, gegebenenfalls mit einem oder mehreren Substituenten, bedeuten, wobei die Substituenten aus der Gruppe ausgewählt sind, die aus Halogen und einem Hydroxyl-, C₁₋₄-Alkyl-, C₁₋₄-Alkoxy-, C₁₋₄-Acyloxy- und C₁₋₄-Carboxylesterrest besteht, oder R₃ und R₄ zur Bildung eines Rings mit vier bis sieben Ringatomen miteinander verbunden sind, X Wasserstoff, einen Hydroxylrest oder Halogen darstellt sowie m und n jeweils unabhängig voneinander einen Wert von 0 bis 5 bedeuten.

2. Salz der Clavulansäure nach Anspruch 1, worin R₁, R₂, R₃ und R₄ jeweils Methyl bedeuten.

3. Salz der Clavulansäure nach Anspruch 1 oder 2, worin X Wasserstoff, n die Zahl 1 und m die Zahl 0 bedeuten.

4. Salz der Clavulansäure nach Anspruch 1 oder 2, worin X einen Hydroxylrest, n die Zahl 1 und m die Zahl 1 bedeuten.

5. Salz der Clavulansäure der Formel IIa nach Anspruch 1, worin R₁, R₂, R₃ und R₄ jeweils Methyl, X Wasserstoff, n die Zahl 1 und m die Zahl 0 bedeuten.

6. Verfahren zum Herstellen eines Salzes nach Anspruch 1, 2, 3 oder 4, worin Clavulansäure in einem organischen Lösungsmittel mit einem Diamin der Formel III 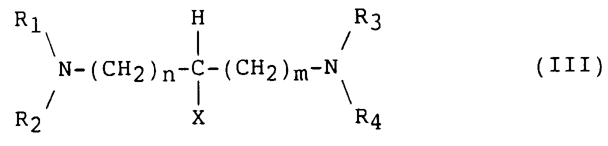 umgesetzt wird, in der R₁, R₂, R₃, R₄, X, m und n wie im Anspruch 1, 2, 3 oder 4 definiert sind.

7. Verfahren nach Anspruch 6, worin die molare Menge des Diamins größer ist als jene der Clavulansäure.

8. Verfahren zur Herstellung eines Salzes gemäß Anspruch 5, worin Clavulansäure mit N,N,N',N'-Tetramethyl-1,2-diaminoethan umgesetzt wird, wobei die molare Menge des N,N,N',N'-Tetramethyl-1,2-diaminoethans größer ist als jene der Clavulansäure.

9. Verfahren zum Herstellen von Clavulansäure oder eines pharmazeutisch akzeptablen Salzes oder Esters hiervon, bei dem ein Salz gemäß Anspruch 1, 2, 3, 4 und 5 in Clavulansäure oder ein pharmazeutisch akzeptables Salz oder einen Ester hiervon umgewandelt wird.

10. Verfahren zum Reinigen von Clavulansäure oder eines pharmazeutisch akzeptablen Salzes oder Esters hiervon mit folgenden Verfahrensstufen:
i) Vorliegen von unreiner Clavulansäure in einem organischen Lösungsmittel mit einem Diamin der Formel III, um ein Salz zu bilden;
ii) Isolieren des in der Stufe i) gebildeten Salzes; und
iii)Umwandeln des isolierten Salzes in Clavulansäure oder in ein pharmazeutisch akzeptables Salz oder einen Ester hiervon.

11. Pharmazeutische Zusammensetzung, enthaltend ein Salz der Clavulansäure gemäß Anspruch 1, 2, 3, 4 oder 5 und ein pharmazeutisch akzeptables Träger- oder Verdünnungsmittel.

12. Verwendung eines Salzes gemäß Anspruch 1, 2, 3, 4 oder 5 als Zwischenprodukt in einem Verfahren zur Herstellung von Clavulansäure oder eines pharmazeutisch akzeptablen Salzes oder Esters hiervon.

## Revendications

1. Sel de l'acide clavulanique de formule (IIa) : 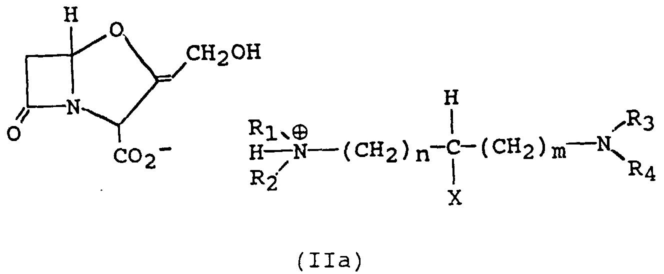 où :
R₁ et R₂ sont chacun un radical alcoyle en C₁₋₈, cycloalcoyle en C₃₋₈ ou (cycloalcoyl en C₃₋₈)alcoyle en C₁₋₈, ayant facultativement un ou plusieurs substituants inertes choisis dans le groupe consistant en halogène, hydroxyle, alcoyle en C₁ à C₄, alcoxy en C₁ à C₄, acyloxy en C₁ à C₄ et carboxyle en C₁ à C₄ estérifié, ou ils sont reliés pour former un cycle de 4 à 7 atomes;
R₃ et R₄ sont chacun, un radical alcoyle en C₁₋₈, cycloalcoyle en C₃₋₈ ou (cycloalcoyl en C₃₋₈)alcoyle en C₁₋₈, ayant facultativement un ou plusieurs substituants inertes choisis dans le groupe consistant en halogène, hydroxyle, alcoyle en C₁ à C₄, alcoxy en C₁ à C₄, acyloxy en C₁ à C₄ et carboxyle en C₁ à C₄ estérifié, ou ils sont reliés pour former un cycle de 4 à 7 atomes;
X est un hydrogène, hydroxyle ou halogène, et m et n sont chacun, indépendamment, 0 à 5.

2. Sel de l'acide clavulanique suivant la revendication 1, dans lequel R₁, R₂, R₃ et R₄ sont méthyle.

3. Sel de l'acide clavulanique suivant la revendication 1 ou 2, dans lequel X est hydrogène, n est 1 et m est 0.

4. Sel de l'acide clavulanique suivant la revendication 1 ou 2, dans lequel X est hydroxyle, n est 1 et m est 1.

5. Sel de l'acide clavulanique de formule (IIa) suivant la revendication 1, dans lequel R₁, R₂, R₃ et R₄ sont méthyle, X est hydrogène, n est 1 et m est 0.

6. Procédé de préparation d'un sel suivant la revendication 1, 2, 3 ou 4, dans lequel on fait réagir l'acide clavulanique avec une diamine (III) : 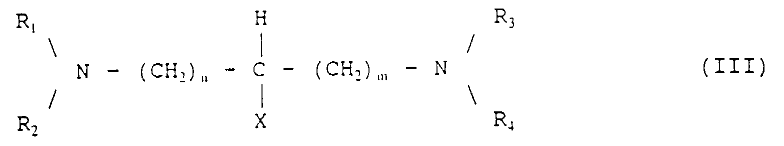 où :
R₁, R₂, R₃, R₄, X, m et n sont tels que définis à la revendication 1, 2, 3 ou 4, dans un solvant organique.

7. Procédé suivant la revendication 6, dans lequel la quantité molaire de la diamine est supérieure à celle de l'acide clavulanique.

8. Procédé de préparation d'un sel suivant la revendication 5, dans lequel on fait réagir l'acide clavulanique avec le N,N,N',N'-tétraméthyl-1,2-diaminoéthane, où la quantité molaire du N,N,N',N'-tétraméthyl-1,2-diaminoéthane est supérieure à celle de l'acide clavulanique.

9. Procédé de préparation d'acide clavulanique ou d'un sel ou ester pharmaceutiquement acceptable de celui-ci, qui comprend la conversion d'un sel suivant les revendications 1, 2, 3, 4 et 5, en acide clavulanique ou en sel ou ester pharmaceutiquement acceptable de celui-ci.

10. Procédé de purification de l'acide clavulanique ou d'un sel ou ester pharmaceutiquement acceptable de celui-ci, le procédé comprend :
i) la mise en commun d'acide clavulanique impur dans un solvant organique et d'une amine de formule (III), pour former un sel de formule (IIa);
ii) l'isolement du sel produit à l'étape i), et
iii) la conversion du sel isolé en acide clavulanique ou en un sel ou ester pharmaceutiquement acceptable de celui-ci.

11. Composition pharmaceutique comprenant un sel de l'acide clavulanique suivant la revendication 1, 2, 3, 4 ou 5 et un excipient ou diluant pharmaceutiquement acceptable.

12. Utilisation d'un sel suivant la revendication 1, 2, 3, 4 ou 5 comme intermédiaire dans un procédé pour la préparation d'acide clavulanique ou d'un sel ou ester pharmaceutiquement acceptable de celui-ci.
